Europäisches Patentamt

European Patent Office

Office européen des brevets

(11) Veröffentlichungsnummer: **0 338 436**

**A2**

(12) **EUROPÄISCHE PATENTANMELDUNG**

(21) Anmeldenummer: 89106626.8

(22) Anmeldetag: 13.04.89

(51) Int. Cl.4: **C07K 15/00 , C12N 5/00 , C12N 15/00 , C12P 21/00 , G01N 33/577 , A61K 39/395 , G01N 33/574 , //(C12P21/00, C12R1:91)**

Patentansprüche für folgende Vertragsstaaten:
ES + GR

(30) Priorität: 22.04.88 DE 3813592

(43) Veröffentlichungstag der Anmeldung:
25.10.89 Patentblatt 89/43

(84) Benannte Vertragsstaaten:
AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Bohuon, Claude, Prof.**
**14 bis Rue Mouton-Duvernet**
**F-75014 Paris(FR)**
Erfinder: **Comoy, E. J., Prof.**
**13 Rue du Hameau**
**F-84240 L'Hay-Les-Roses(FR)**

(54) **Monoklonale Antikörper gegen das Neuropeptid Y, Verfahren zu ihrer Herstellung sowie ihre Verwendung.**

(57) Die Erfindung betrifft monoklonale Antikörper (MAK) gegen das Neuropeptid Y (NPY). Es wurden 4 MAKs gefunden die gegen folgende Regionen des NPY gerichtet sind:

```
MAK     NPY 04     gegen Aminosäuresequenz     1 - 12 des NPY
MAK     NPY 02     gegen Aminosäuresequenz     8 - 18 des NPY
MAK     NPY 03     gegen Aminosäuresequenz    27 - 36 des NPY
MAK     NPY 05     gegen Aminosäuresequenz    32 - 36 des NPY*

    * einschließlich N-Terminus
```

Weiterhin betrifft die Erfindung die Verwendung der MAKs NPY 02 -NPY 05, insbesondere als Diagnostikum zur Bestimmung von humanem NPY (h NPY).

_Fig. 1_

NPY 02

Legende:
- hNPY11-24
- hNPY1-36
- pPYY
- ANDERE PEPTIDE

% INHIBITION

PEPTID LOG (mol/l)

EP 0 338 436 A2

## Monoklonale Antikörper gegen das Neuropeptid Y, Verfahren zu ihrer Herstellung sowie ihre Verwendung

Humanes Neuropeptid Y (hNPY) ist ein Peptid mit 36 Aminosäuren (Tatemoto, K. Neuropeptide Y: The complete amino acid sequence of the brain peptide, Proc. Natl. Acad. Sci. USA 79: 5485-5489, 1982), das im Gewebe und Serum von Phaeochromocytom- und Ganglioneuroblastom-Patienten nachgewiesen wurde. NPY wirkt antagonistisch und wird für die Freisetzung von Noradrenalin verantwortlich gemacht. Interessant erscheint NPY (hNPY) als Tumormarker für steroidhormon-produzierende Tumore, Schilddrüsenkarzinome und neuroendocrine Tumore (Neuroblastom).

NPY zeigt 50 % bzw. 70 % sequenzielle Analogie zu PP (pancreatic polypeptide) und zum PYY (peptide YY).

Ein immunoradiometrischer Assay (IRMA) zur Bestimmung von NPY in Plasma ist bereits beschrieben (Corder, R., Lowry, P.J., Peptides 6 (6), 1195 - 1200, 1985). Dabei werden polyklonale Antikörper bzw. Antikörperseren, die durch Immunisierung von Schafen bzw. Kaninchen mit NPY bzw. NPY-Teilsequenzen erhalten wurden, verwendet. Der markierte Sandwichkomplex wird mit einem festphasengebundenen Antikörper, der gegen die Fc-Regionen eines im Assay verwendeten Antikörpers gerichtet ist, abgetrennt.

Die vorliegende Erfindung betrifft nun monoklonale Antikörper, die gegen folgende Regionen des NPY gerichtet sind:

```
MAK (NPY 04) gegen Aminosäure-Sequenz  1 - 12 des NPY
MAK (NPY 02) gegen Aminosäure-Sequenz  8 - 18 des NPY
MAK (NPY 03) gegen Aminosäure-Sequenz 27 - 36 des NPY
MAK (NPY 05) gegen Aminosäure-Sequenz 32 - 36 des NPY*
                  *einschließlich N-Terminus
```

Diese monoklonalen Antikörper können als Diagnostikum, Wirkstoff oder Wirkstoffträger verwendet werden. Vier Hybridomazellklone konnten gewonnen werden, deren sezernierte MAKs (NPY 02 - NPY 05) charakterisiert wurden.

Die Identifikation der monoklonalen Antikörper wurde in einem ELISA-Verfahren durchgeführt, wobei Kaninchen-Antikörper benutzt wurden, die gegen die verschiedenen Immunglobulin-Subklassen gerichtet waren (Nordic). Die Messung der Affinitätskonstanten erfolgte durch Inkubation der MAKs mit radioaktiv markiertem NPY in Gegenwart von steigenden Konzentrationen des unmarkierten Gesamtpeptids nach einem schnellen RIA-Verfahren. Die so erhaltenen RIA-Bindungsdaten wurden zur Ermittlung der Affinitätskonstanten ($K_a$) nach einer Scatchard-Plot-Analyse herangezogen.

Zum Test der Anti-hNPY-Antikörper in den verschiedenen Schritten der MAK-Herstellung (Mausserum, Hybridomazellen, Zellüberstände der klonierten Zellen, Aszitesflüssigkeiten) wurde ein schneller RIA benutzt.

Dabei wurden 10 bis 100 $\mu$l Medium 2 Stunden bei 20° C mit 500 $\mu$l Phosphatpuffer (50 mmol, pH 7,5) inkubiert, wobei der Phosphatpuffer 20 mmol EDTA, 0,3 % BSA (bovine serum albumin), 0,005 % Aprotinin und mit $J^{125}$ markiertes NPY ($3.10^3$ cpm; specific activity: 73 TBq/mmol;) enthielt. Durch Hinzugabe von 500 $\mu$l eines Phosphatpuffers, der 0,02 % Gelatine, 1,6 % Aktivkohle und 0,16 % Dextran enthielt, wurde die Inkubation gestoppt. Nach Schütteln (10 sec) und Zentrifugation wurde die radioaktive Strahlung sowohl in den Überständen (B = gebundenes NPY) als auch im Niederschlag (F = freies NPY) mit einem Zählrohr gemessen. Der Quotient aus gebundenem und gebundenem plus freiem Anteil ergab die prozentuale Bindung von NPY an die MAKs.

Die Lokalisation der Epitope, die von den verschiedenen MAKs erkannt werden, erfolgte in einem kompetitiven Inhibitionsassay mit synthetisch hergestellten hNPY-Fragmenten und mit den NPY-verwandten Peptiden hPP (human pancreatic polypeptide), bPP (bovine pancreatic polypeptide) und pPYY (porcine peptide YY). Diese Bindungsdaten wurden ebenfalls in einem RIA gemessen.

Im folgenden ist die weitere Ausgestaltung der Erfindung beschrieben.

Die Herstellung von hNPY und seiner Teilsequenzen (1-12, 11-18, 11-24, 23-36, 27-36 und 27-36 (C-terminusfrei)) erfolgte durch Festphasen-Merrifield-Synthese (Merrifield, R.B., Solid phase peptide synthesis. I. The synthesis of a tetrapeptide, J. Am. Chem. Soc. 85, 2149, (1963)). Die Synthesen wurden mit einem "automatic synthesizer" (Applied Biosystems, Model 430A, Foster City, CA) durchgeführt. Die Abspaltung der Peptide von dem Trägerpolymer erfolgt mit Fluorwasserstoffsäure. Anschließend wurden die Peptide durch Gelfiltration über Bio-Gel P4 (Bio-Rad, Richmond, CA) gereinigt. Die Reinheit wurde mittels Reversed-Phase-LC überprüft und die Aminosäurezusammensetzung an einem LKB Alpha Analyser bestimmt.

Die verwandten Peptide hPP, bPP und pPYY sind käuflich erhältlich (beispielsweise Sigma Chemicals Co., St. Louis, Mo).

Zur Herstellung der monoklonalen Antikörper wurde als Immunogen entweder hNPY selbst (für die MAKs NPY 02 -NPY 04) oder die Teilaminosäuresequenz 27-36 (für den MAK NPY 05), jeweils gekoppelt an KLH (keyhole limpet haemocyanin, SIGMA), mit Glutaraldehyd als Kopplungsagenz, verwendet.

Die erste Immunisierung der 6 Wochen alten Mäuse erfolgte durch Subcutaninjektion von 25 µg des oben beschriebenen Immunogens in komplettem Freund'schen Adjuvans. Die darauf folgenden Injektionen wurden monatlich mit der gleichen Konzentration des Immunogens in komplettem Freund'schen Adjuvans durchgeführt. Jeweils vor diesen Folgeimmunisierungen wurde der Antikörpergehalt im Mausserum nach der oben beschrieben schnellen RIA-Methode bestimmt. Bei einem positiven Befund (d.h. 60 % Bindung oder mehr mit dem auf 50 % verdünnten Mausserum) wurden noch zwei Immunogen-Dosen verabreicht; einmal 9 Tage (i.p.) und einmal 3 Tage (i.v.) vor der Fusion.

Die Zellfusion, Hybridoma Selektion, Klonierung und Aszites Produktion wurden nach der Methode, die von Bidart et al. (Identification of epitopes associated with hCG and hCG carboxyl terminus by monoclonal antibodies produced against a synthetic peptide, J. Immunol. 134, 457 (1985)) beschrieben wurde, durchgeführt. Dabei wurden die Mäusemilzzellen mit NS 1 Myelom-Zellen durch Inkubation in Anwesenheit von 40 % (w/v) Polyethylenglykol (Molekulargew.: 1000) fusioniert. Nach einer Kultivierungszeit von zwei Wochen wurden die Zellüberstände in den Screening-RIA (Verfahren wie oben beschrieben) zwecks Gewinnung von Anti-hNPY-Antikörper überführt. Zellen aus 75 (71 aus den Immunisierungen mit vollständigem hNPY, 4 aus den Immunisierungen mit dem 27-36-Subpeptid) RIA-positiven Wells (Vertiefungen einer Mikrotiterplatte) wurden kloniert und jeweils $2 \cdot 10^6$ Zellen von ausgewählten 4 Hybridomas (3 resultierend aus hNPY, 1 resultierend aus dem 27-36-Subpeptid) wurden dann nackten Mäusen i.p. injiziert. Zwei Wochen nach der Injektion wurde die Aszitesflüssigkeit entnommen.

Die so erhaltenen 4 monoklonalen Antikörper NPY 02-NPY 05 (MAK NPY 05 resultierend aus der Immunisierung mit dem 27-36-Subpeptid) wurden durch Präzipitation mit 50 %iger Ammoniumsulfat-Lösung von der Maus-Aszitesflüssigkeit getrennt. Anschließend erfolgte die Reinigung der monoklonalen Antikörper entweder durch Protein-A-Sepharose- Affinitäts-Chromatographie (beschrieben von EY et al., Isolation of pure IgG1, IgG2a und IgG2b immunoglobulines from mouse serum using protein A sepharose, Immunochemistry, 15, 429, (1978)) (im Fall des igG2-Antikörpers NPY 05) oder durch DEAS-Sephacel-Chromatographie mit anschließender Protein A Prozedur (im Fall der Antikörper NPY 02-04). Die gereinigten monoklonalen Antikörper wurden dann nach der Iodogenmethode (Fraker, P.SJ, Speck, J.C., Protein and cell membrane iodination using a sparingly soluble chloroamide, Biochem. Biophys. Res. Commun., 80, 849, (1977)) mit $J^{125}$ markiert (spezifische Aktivität: 450-600 KBq/µg Immunoglobulin). Vor dem Einsatz wurden die markierten monoklonalen Antikörper mit PBS, welches 20 mmol EDTA, 10 mmol Barbital, 1 % BSA und 50 UI/1 Aprotinin enthielt, versetzt.

In Tabelle 1 sind die wesentlichen Charakteristika der monoklonalen Antikörper NPY 02-NPY 05 zusammengefaßt.

## Tabelle 1

| MAK | Immunglobulin Unterklasse | erkanntes Epitop auf hNPY | Affinitätskonst. Ka $[\text{l} \cdot \text{mol}^{-1}]$ |
|---|---|---|---|
| NPY 02 | IgG 1 | 8 - 18 | $5{,}5 \cdot 10^{10}$ |
| NPY 03 | IgG 2ab | 27 - 36 | $6{,}7 \cdot 10^9$ |
| NPY 04 | IgG 3 | 1 - 12 | $3{,}8 \cdot 10^8$ |
| NPY 05 | IgG 2ab | 32 - 36 | $2{,}5 \cdot 10^{10}$ |

Die von den monoklonalen Antikörpern NPY 02-NPY 05 erkannten Epitope auf hNPY wurden in einem

3

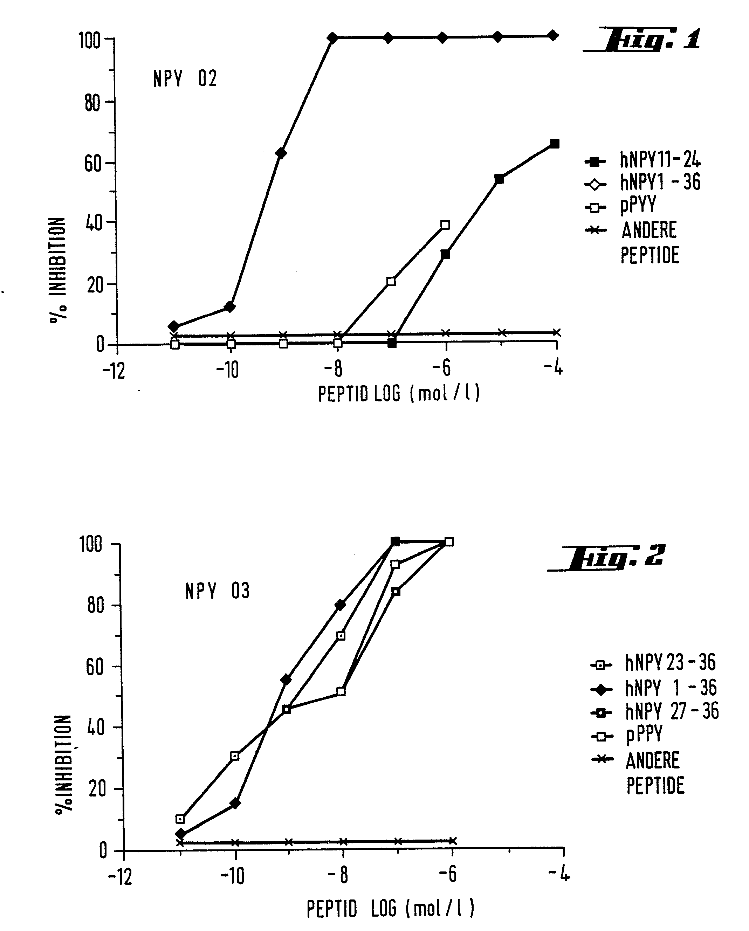

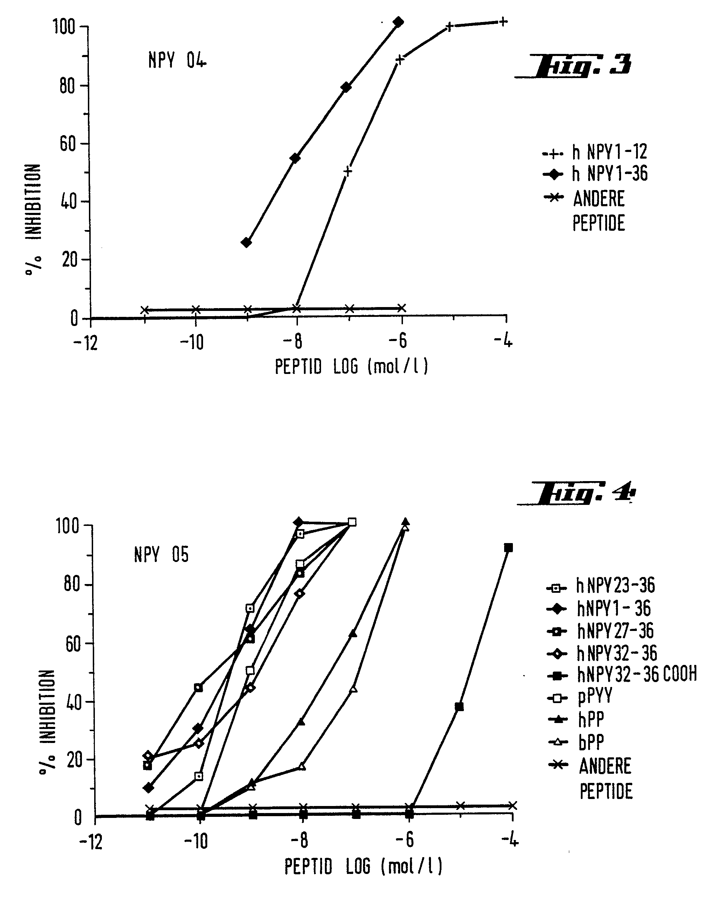

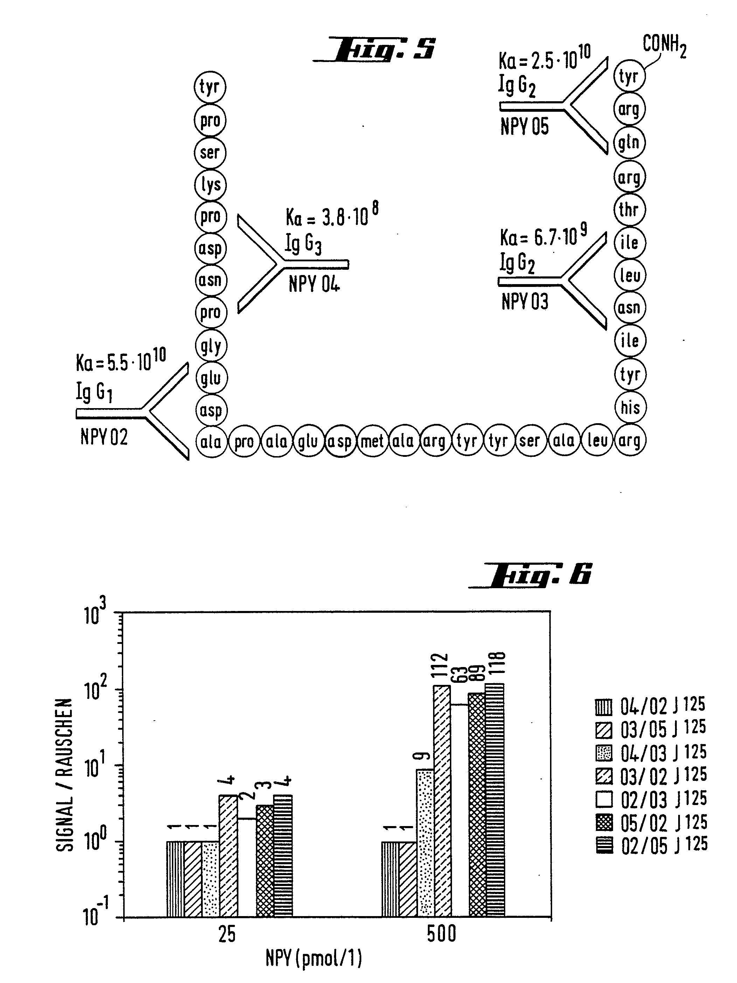

Inhibitionstest mit verschiedenen hNPY-Teilsequenzen und hNPY-verwandten Peptiden ermittelt. Die Figuren 1 bis 4 zeigen die prozentuale Inhibition der vier monoklonalen Antikörper, aufgetragen gegen die Konzentration von verschiedenen hNPY-Teilsequenzen bzw. hNPY-verwandten Peptiden. Die IC 50-Werte ( = notwendige Konzentration für eine 50%ige Inhibition) der monoklonalen Antikörper NPY 02-NPY 05 sind in Tabelle 2 angegeben.

## Tabelle 2 (IC 50-Werte [mol])

|  | NPY 02 | NPY 03 | NPY 04 | NPY 05 |
|---|---|---|---|---|
| hNPY 1-12 |  |  | $9 \cdot 10^{-9}$ |  |
| pPYY 1-12 |  |  | $1 \cdot 10^{-7}$ |  |
| hNPY 11-24 | $1 \cdot 10^{-5}$ |  |  |  |
| pPYY 11-24 | $6 \cdot 10^{-6}$ |  |  |  |
| hNPY 23-36 |  | $7 \cdot 10^{-10}$ |  | $4 \cdot 10^{-10}$ |
| hNPY 27-36 |  | $2 \cdot 10^{-9}$ |  | $5 \cdot 19^{-10}$ |
| hNPY 32-36 |  | $> 10^{-4}$ |  | $1 \cdot 10^{-9}$ |
| pPYY 23-36 |  | $1 \cdot 10^{-8}$ |  | $1 \cdot 10^{-9}$ |
| hPP 23-36 |  | $> 10^{-4}$ |  | $4 \cdot 10^{-8}$ |
| hNPY 32-36-COOH |  |  |  | $2 \cdot 10^{-5}$ |

Von dem monoklonalen Antikörper NPY 02 wird allein das 11-24-Fragment von hNPY und pPYY erkannt. Bindungen zu anderen Fragmenten oder Peptiden treten nicht auf. Diese Resultate legen nahe, daß die antigene Determinante zwischen den Aminosäuren 8 und 24 lokalisiert ist, da pPYY und hNPY in diesem Bereich nur die Sequenz 8-12 gemeinsam haben. Da die 18-24-Teilsequenz und die 1-12-Teilsequenz nicht an den monoklonalen Antikörper NPY 02 bindet, verkürzt sich die antigene Determinante auf die Aminosäuresequenz 8-18, wahrscheinlich 10-15.

Der monoklonale Antikörper NPY 05 zeigt eine hohe Affinität für pPYY, hPP, bPP und für die C-terminale amidische Region. Die 32-36-Teilsequenz mit freier COOH-Gruppe wird nur schwach erkannt (IC 50 = $2 \bullet 10^{-5}$ mol). Da zum einen der monoklonale Antikörper NPY 05 durch Immunisierung mit der 27-36-Teilsequenz erhalten wurde und andererseits die Bindung von $J^{125}$-NPY an den Antikörper sowohl durch das 32-36-Subpeptid, als auch durch das gesamte hNPY inhibiert wird, liegt das von NPY 05 erkannte Epitop sicherlich auf den letzten fünf Aminosäuren von hNPY. Die starke Abnahme des IC 50-Wertes im Fall der Erkennung des 32-36-COOH-Fragments (IC 50 = $2 \bullet 10^{-5}$ mol) zeigt die Bedeutung der Amidgruppe in dieser Epitop-Bindungsregion an. Figure 5 zeigt hNPY mit den vier gebundenen monoklonalen Antikörpern NPY 02-NPY 05.

Für die Entwicklung eines IRMA ("Sandwich Assay") zur Bestimmung der hNPY-Konzentration in Seren wurden die monoklonalen Antikörper NPY 02 bis NPY 05 jeweils einmal an die Festphase eines Polystyrol-Teströhrchens gebunden und zum anderen mit $J^{125}$ radioaktiv markiert. Das Signal-Rausch-Verhältnis von verschiedenen Antikörperpaaren in Abhängigkeit von zwei verschiedenen NPY-Konzentrationen (25 pmol/l und 500 pmol/l) ist in Figur 6 gezeigt. Die Kombination MAK NPY 02 auf der Festphase und MAK NPY 05 radioaktiv markiert (S/R bei 500 pmol/l NPY = 118; siehe Figur 6) hat sich dabei als günstig erwiesen. Die monoklonalen Antikörper NPY 03 und NPY 04 scheinen interessant für die Bestimmung von Precursoren von hNPY, insbesondere solchen, mit längerer Aminosäuresequenz.

Die beiden monoklonalen Antikörper NPY 02 und NPY 05 wurden, nicht zuletzt aufgrund ihrer guten Affinitätskonstanten und aufgrund der Lage der Bindungsstellen auf hNPY, für die Entwicklung eines IRMA für hNPY ausgewählt. Für den "Sandwich-Assay" wurde der MAK NPY 02 an der Festphase von Polystyrol-Teströhrchen gebunden und der MAK NPY 05 radioaktiv mit $J^{125}$ markiert (150$\bullet 10^3$ cpm, spec. activity = 450-600 KBq/µg). 300 µl eines NPY-Standards (0-100 fmol/ml) bzw. eines Patienten-Testserums wurde in die beschichteten Teströhrchen gegeben. Anschließend wurden 50 µl des markierten MAKs hinzugegeben (Inkubationsdauer: 16 Stunden bei 4°C unter leichtem Schütteln). Nach einem Waschschritt (3 mal 1 ml entionisiertes Wasser, welches 0,3 % Tween 20 enthielt) wurde die an die Festphase gebundene Radioakti-

4

vität gemessen. Durch Mittelwertbildung von 2 Proben mit gleicher Standard-NPY-Konzentration wurde das Signal-Rausch-Verhältnis bestimmt. Die Mittelwerte wurden zur Erstellung einer Eichkurve herangezogen.

Die Empfindlichkeit dieses Assays lag bei 0,5 fmol/ml hNPY. Kreuzreaktionen der verwendeten monoklonalen Antikörper mit PP bzw. PYY konnten bis zu einer PP- bzw. PYY-Konzentration von 10 nmol/ml nicht festgestellt werden.

In 217 Plasmaproben von gesunden Patienten zwischen 18 und 55 Jahren wurde die hNPY-Konzentration mit dem oben beschriebenen Sandwich-Assay bestimmt. 95 % der Proben zeigten hNPY-Konzentrationen unterhalb 5 fmol/ml. Diese Konzentration wurde als obere physiologische Grenze angenommen. Von den restlichen Proben mit einer hNPY-Konzentration oberhalb 5 fmol/ml befanden sich 6 noch unterhalb 10 fmol/ml, die übrigen besaßen hNPY-Konzentrationen von maximal 30 fmol/ml. Bei Patienten mit neuroendocrinen Tumoren (56 Plasmaproben wurden gemessen) lag die hNPY-Konzentration signifikant höher (bei Phaeochromocytom und Neuroblastom). Die höchsten Plasma-hNPY-Werte wurden beim Patienten mit malignem Phaeocromocytom beobachtet.

## Ansprüche

1. Monoklonale Antikörper (MAK) NPY 02, NPY 03, NPY 04 und NPY 05, die mit NPY reagieren.

2. Hybridome, die die MAK nach Anspruch 1 sezernieren.

3. Verfahren zur Erzeugung der Hybridome nach Anspruch 2, dadurch gekennzeichnet, daß ein Säugetier mit NPY immunisiert wird, Milzzellen aus einem solchen Tier entnommen, mit NS1 Myelomzellen fusioniert und die Hybridome auf Sezernierung von MAK gegen NPY selektioniert werden.

4. Verwendung eines MAK nach Anspruch 1 in einem Diagnostikum.

5. Verwendung eines MAK nach Anspruch 1 als Träger für einen pharmazeutischen Wirkstoff.

6. Verwendung eines MAK nach Anspruch 1 als Therapeutikum.

7. Verwendung eines monoklonalen Antikörpers nach Anspruch 1 in markierter Form als Diagnostikum für die Analyse von Geweben und Körperflüssigkeiten und für die Radioimmunszintigraphie.

8. Verwendung eines monoklonalen Antikörpers nach Anspruch 1 in markierter Form als Therapeutikum für die Radioimmuntherapie oder für die Chemoimmuntherapie.

9. Diagnostikum zur Bestimmung von hNPY, gekennzeichnet durch
- einen festphasengebundenen MAK, ausgewählt aus NPY 02, NPY 03, NPY 04, NPY 05 und
- einem radioaktiv markierten MAK, ausgewählt aus NPY 02, NPY 03, NPY 04, NPY 05,
wobei der festphasengebundene MAK nicht mit dem radioaktiv markierten MAK identisch ist.

10. Diagnostikum nach Anspruch 9, dadurch gekennzeichnet, daß der festphasengebundene MAK der MAK NPY 02 ist, und der radioaktiv markierte MAK der MAK NPY 05 ist.


· Patentansprüche für folgende Vertragsstaaten: ES, GR

1. Verfahren zur Herstellung der monoklonalen Antikörper (MAK) NPY 02, NPY 03, NPY 04 und NPY 05, die mit NPY reagieren, durch Selektionierung von Hybridomen, die die MAKs NPY 02, NPY 03, NPY 04 und NPY 05 sezernieren.

2. Verfahren zur Erzeugung von Hybridomen, die die MAKs NPY 02, NPY 03, NPY 04 und NPY 05 szernieren, dadurch gekennzeichnet, daß ein Säugetier mit NPY immunisiert wird,Milzzellen aus einem solchen Tier entnommen, mit NS1 Myelomzellen fusioniert und die Hybridome auf Sezernierung von MAK gegen NPY selektioniert werden.

3. Verwendung eines MAK nach Anspruch 1 in einem Diagnostikum.

4. Verwendung eines MAK nach Anspruch 1 als Träger für einen pharmazeutischen Wirkstoff.

5. Verwendung eines MAK nach Anspruch 1 als Therapeutikum.

6. Verwendung eines monoklonalen Antikörpers nach Anspruch 1 in markierter Form als Diagnostikum für die Analyse von Geweben und Körperflüssigkeiten und für die Radioimmunszintigraphie.

7. Verwendung eines monoklonalen Antikörpers nach Anspruch 1 in markierter Form als Therapeutikum für die Radioimmuntherapie oder für die Chemoimmuntherapie.

8. Diagnostikum zur Bestimmung von hNPY, gekennzeichnet durch
- einen festphasengebundenen MAK, ausgewählt aus NPY 02, NPY 03, NPY 04, NPY 05 und
- einem radioaktiv markierten MAK, ausgewählt aus NPY 02, NPY 03, NPY 04, NPY 05,
wobei der festphasengebundene MAK nicht mit dem radioaktiv markierten MAK identisch ist.

9. Diagnostikum nach Anspruch 9, dadurch gekennzeichnet, daß der festphasengebundene MAK der MAK NPY 02 ist, und der radioaktiv markierte MAK der MAK NPY 05 ist.

Fig. 1

Fig. 2

Fig. 3

NPY 04

% INHIBITION

PEPTID LOG (mol/l)

-·+·- h NPY 1-12
-◆- h NPY 1-36
-✳- ANDERE
PEPTIDE

Fig. 4

NPY 05

% INHIBITION

PEPTID LOG (mol/l)

-⊡- h NPY 23-36
-◆- h NPY 1-36
-⊟- h NPY 27-36
-◇- h NPY 32-36
-■- h NPY 32-36 COOH
-□- p PYY
-▲- h PP
-△- b PP
-✳- ANDERE
PEPTIDE

Fig. 5

Fig. 6